# EUROPEAN PATENT APPLICATION

(11) **EP 4 792 841 A1**
(43) Date of publication of application: **19.08.2026**
(21) Application number: 24876058.9
(22) Date of filing: 06.06.2024
(51) Int. Cl.: A61L 27/40, A61L 27/54, A61F 2/02

(54) **BIODEGRADABLE FILM HAVING THREE-DIMENSIONAL ORDERED POROUS STRUCTURE, AND PREPARATION METHOD THEREFOR AND USE THEREOF**

(30) Priority: 13.10.2023 CN 202311322485
(71) Applicant: Hangzhou Yinsheng Medical Technology Co., Ltd., Hangzhou, Zhejiang 311106 (CN)
(72) Inventor: WEI, Xiaoqin, Hangzhou, Zhejiang 311106 (CN); ZHANG, Jingbo, Hangzhou, Zhejiang 311106 (CN)
(74) Representative: Gong, Jinping
(86) International application number: PCT/CN2024/097686
(87) International publication number: WO 2025/077232

(57) **Abstract**

The invention provides a biodegradable membrane with a three-dimensional ordered porous structure, a preparation method therefor and an application thereof. The biodegradable membrane has good mechanical properties, with upper and lower surfaces capable of constructing the same or different ordered porous micro-nano structures, thereby enabling the repair of the same or different tissues. Meanwhile, a degradation time of the corresponding structural layer can be controlled by structural differences and material selection to adapt to a repair cycle of different tissues and improve repair effects. In addition, an intermediate disordered fiber layer is disposed to not only provide a scaffold for tissue engineering with selective permeability but also ensures that the repair of a first ordered layer and a second ordered layer does not interfere with each other, thus expanding the applicability of the scaffold for tissue engineering. The preparation method of the invention adopts a near-field direct-write 3D printing method. By adjusting printing parameters of different structural layers, the structure of each layer can be controlled to give a product the expected performance. The preparation method is simple, convenient to operate, and easy to implement.

## Description

### FIELD OF THE INVENTION

The invention belongs to the technical field of medical tissue engineering, in particular, to a biodegradable membrane with a three-dimensional ordered porous structure, a preparation method therefor and an application thereof.

### BACKGROUND OF THE INVENTION

Tissue engineering is a newly emerging discipline in recent years. It mainly refers to the science of applying the principles and technologies of life sciences and engineering to study and develop biological substitutes for repairing, maintaining, and promoting the function and morphology of various tissues or organs in the human body after damage. As the effectiveness of tissue engineering is continuously verified through research and application, its application in the repair of damaged human tissues is attracting increasing attention from researchers.

An ideal scaffold for tissue engineering should not only have excellent biocompatibility, but also a three-dimensional structure similar to the extracellular matrix of different human tissues, to facilitate cell adhesion, guide the directional growth of cells, and thus better promote autologous tissue regeneration.

Scaffolds for tissue engineering manufactured using existing traditional technologies such as electrospinning and freeze-drying cannot form an ordered three-dimensional porous structure, thus failing to efficiently promote cell adhesion and growth on their surface. Near-field direct-write 3D printing is a unique technology that can manufacture ordered structures. Based on the advantage of being able to prepare ordered three-dimensional structures, this technology has been widely used in the field of tissue engineering. However, the scaffolds for tissue engineering currently manufactured by near-field direct-write are all designed for homogeneous structures, and are mostly continuously-stacked disordered structures or have only one side being an ordered structure. Such structures are designed for homogeneous and symmetrical structures and cannot achieve synchronous repair of different locations in the body. For example, when guided bone regeneration surgery is performed in the oral cavity, there is a problem that the gums cannot close. Either tension-reduction sutures or autologous gingival transplantation can be used for repair, but both of these methods are prone to postoperative pain and discomfort, which will increase the risk of postoperative infection and produce less aesthetically pleasing results.

### BRIEF SUMMARY OF THE DISCLOSURE

In order to solve the problems in the prior art, the invention provides a biodegradable membrane with a three-dimensional ordered porous structure and a preparation method therefor. The biodegradable membrane has good mechanical properties, with upper and lower surfaces capable of constructing the same or different ordered micro-nano structures, thereby enabling the repair of the same or different tissues. Meanwhile, the degradation cycle, mechanical properties, etc. of the corresponding structural layers can be controlled by structural differences and material selection to adapt to repair cycles and mechanical requirements of different types of tissues and improve repair effects. In addition, an intermediate disordered fiber layer is disposed to not only provide a scaffold for tissue engineering with selective permeability but also ensures that the repair of a first ordered layer and a second ordered layer does not interfere with each other, thus expanding the applicability of the scaffold for tissue engineering. The preparation method of the invention adopts a near-field direct-write 3D printing method. By adjusting printing parameters of different structural layers, the structure of each layer can be controlled to give a product the expected performance. The preparation method is simple, convenient to operate, and easy to implement.

A biodegradable membrane with a three-dimensional ordered porous structure includes an intermediate disordered fiber layer formed by disorderly stacking helical fibers, and a first ordered fiber layer and a second ordered fiber layer disposed on both sides of the intermediate disordered fiber layer, the first and second ordered fiber layers being each independently formed by stacking single or multiple layers of linear fibers and having an ordered structure;
a first ordered fiber layer material, an intermediate disordered fiber layer material, and a second ordered fiber layer material are each independently selected from a biodegradable material.

The first ordered fiber layer and the second ordered fiber layer are each independently formed by stacking single or multiple layers of linear fibers. The first ordered fiber layer and the second ordered fiber layer may be selected to have the same structure or different structures as needed. The ordered structure may be a regular grid structure, and the grid may be of a square shape, a rhomboid shape, a triangular shape, etc.

Preferably, the biodegradable material is one or more copolymers or mixtures of polylactic acid, polyglycolic acid, polycaprolactone, polylactic-co-hydroxyacetic acid copolymer, polyethylene glycol, polyurethane, polyhydroxybutyrate, polyhydroxyvalerate, polydioxanone, polytrimethylene carbonate, polybutylene succinate, chitosan and derivatives thereof, carboxymethyl cellulose, gelatin, hyaluronic acid, collagen, alginate, gum, whey protein and derivatives thereof.

Preferably, a fiber spacing of the first ordered fiber layer and the second ordered fiber layer are each independently selected from 1 to 500 µm.

Preferably, a diameter of the fiber is 0.1 to 200 µm.

Further preferably, diameters of the first ordered fiber layer and the second ordered fiber layer are each independently selected from 2 to 50 µm.

Further preferably, a diameter of the intermediate disordered fiber layer is 1 to 20 µm.

A preparation method for the biodegradable membrane with a three-dimensional ordered porous structure according to any one of the above descriptions includes: using near-field direct-write 3D printing technology to print the first ordered fiber layer and the second ordered fiber layer with parameters of a nozzle-substrate distance of 0.5~5mm, an extrusion pressure of 0.1KPa~1MPa and a voltage of 1~6KV;
printing the intermediate disordered fiber layer with parameters of the nozzle-substrate distance of 1~50mm, the extrusion pressure of 0.5~50KPa and the voltage of 1~30KV.

For the preparation method of the invention, with near-field direct-write 3D printing technology, by controlling printing parameters (such as nozzle-substrate distance, extrusion pressure, voltage, etc.), the surface tension of the material generates a Taylor cone and forms a "catenary model" effect, thereby forming controllable linear fibers and printing out a set ordered fiber structure. Meanwhile, by controlling the temperature of the printing platform, the linear fibers are crossed, adhered, and stacked, further improving the uniformity and stacking stability of the ordered structure.

When the disordered fiber layers are printed, by controlling printing parameters (such as nozzle-substrate distance, extrusion pressure, voltage, etc.), the "catenary"-like effect is created during printing, which causes the formation of curved helical fibers that cross-stack and form a dense disordered barrier structure, i.e., the intermediate disordered fiber layer. Meanwhile, by controlling the temperature of the printing platform and attracting the ordered fibers at the bottom, the density of the disordered fiber layer is increased to reduce micropore size and membrane porosity, and the selective permeability of the intermediate disordered fiber layer is achieved (which can block cells from passing through while allowing small molecules such as gases, tissue fluids and cytokines to pass through). Compared to the ordered fiber layer, the intermediate disordered fiber layer is formed by disorderly stacking the helical fibers with smaller pores, finer fiber diameters, and a denser arrangement.

Preferably, when the first ordered fiber layer and the second ordered fiber layer are printed, the nozzle-substrate distance is 1~3mm, the extrusion pressure is 0.1~50KPa, and the voltage is 2~5KV.

Preferably, when the intermediate disordered fiber layer is printed, the nozzle-substrate distance is 5~25mm, the extrusion pressure is 1~20KPa and the voltage is 8~20KV.

Preferably, the method includes printing using a continuous printing method, which includes:
sequentially printing the first ordered fiber layer, the intermediate disordered fiber layer, and the second ordered fiber layer on a substrate to obtain the biodegradable membrane with a three-dimensional ordered porous structure.

Specifically, using near-field direct-write 3D printing technology, the ordered structures are printed on smooth substrates, and by overcoming the "catenary"-like effect (adjusting printing process parameters), controllable linear fibers are formed, while controlling the spatial arrangement of the fibers to form the linear ordered structure (the first ordered fiber layer); using the first ordered fiber layer as a substrate, the fiber morphology becomes curved and helical during the printing process by utilizing a "catenary"-like effect (achieved by adjusting the printing process parameters), so that the fibers are randomly stacked on the first ordered fiber layer to form a dense barrier layer, i.e., the intermediate disordered fiber layer; then, using the ordered-disordered (the first ordered fiber layer + the intermediate disordered fiber layer) obtained above as a substrate, the ordered structure is printed, and by overcoming the "catenary"-like effect (adjusting the printing process parameters), the spatial arrangement of fibers is controlled to form the linear ordered structure, while obtaining the second ordered fiber layer on the intermediate disordered fiber layer for further obtaining the biodegradable membrane with a three-dimensional ordered porous structure.

Preferably, the method includes printing using a thermal bonding method, which specifically includes steps of:
sequentially printing a disordered helical fiber layer and the first ordered fiber layer on a substrate to obtain a first composite layer;
sequentially printing the intermediate disordered fiber layer and the second ordered fiber layer on another substrate to obtain a second composite layer;
thermally bonding the disordered helical fiber layer of the first composite layer and the intermediate disordered fiber layer of the second composite layer to obtain the biodegradable membrane with a three-dimensional ordered porous structure;
wherein when the disordered helical fiber layer is printed, the nozzle-substrate distance is controlled to be 1~50mm, the extrusion pressure to be 0.5~50KPa, and the voltage to be 1~30KV.

Further preferably, when the disordered helical fiber layer is printed, the nozzle-substrate distance is 5~25mm, the extrusion pressure is 1~20KPa and the voltage is 8~20KV.

Preferably, the thermal bonding temperature is 0.2 to 0.9 times a melting point of a material with a lower melting point among the intermediate disordered fiber layer material and the disordered helical fiber layer material. Further preferably, the thermal bonding temperature is 0.4 to 0.9 times the melting point.

Preferably, during the thermal bonding process, a certain weight may be placed on top portions of the first composite layer and the second composite layer to ensure a tight bond.

Preferably, a fiber diameter of the disordered helical fiber layer is 0.01 to 1 times the fiber diameter of the first ordered fiber layer, with a fiber path being the same as that of the first ordered fiber layer.

The disordered helical fiber layer is formed by disorderly stacking one or more layers of helical fibers.

Preferably, a roughness of the substrate is *Rₐ*≤1.6µm. For the continuous printing method, when a bottom surface of the ordered layer comes into contact with the substrate, the bottom surface of the ordered layer is easier adhered to the smooth substrate; therefore, selecting a substrate with a roughness of *Rₐ*≤1.6µm further ensures the flatness and stacking of the ordered layer on the bottom. For the thermal bonding method, the disordered layer is first printed onto the substrate, which also makes a bottom surface of the disordered layer smooth, and the helical fibers will spread out so that an area of the bottom is increased to facilitate the thermal bonding.

Preferably, the substrate is selected from one of glass plates, monocrystalline silicon wafers, polytetrafluoroethylene, nylon, polyurethane, etc.

Preferably, during printing, a temperature of the substrate is controlled to be 0.3 to 0.9 times a melting point of a current printing material. By controlling the temperature of the substrate to be close to the melting point of the material, a cooling rate of the fiber is slowed down, allowing the fiber to be fixed on the substrate.

The preparation method of the invention may customize scaffolds with different three-dimensional ordered structural surfaces according to the needs of different tissues, thereby better promoting the repair of different tissues.

The invention further discloses an application of the biodegradable membrane with a three-dimensional ordered porous structure according to any one of the above descriptions as a scaffold for tissue engineering.

Compared with the conventional art, the invention has the following beneficial effects.
1. The scaffold for tissue engineering constructed by the invention has a fine three-dimensional ordered structure on a surface thereof, and with adjustable specific surface area and porosity, cell adhesion, growth and migration may be more effectively promoted, while facilitating the exchange of nutrients and metabolites around the tissue. Further, the upper and lower surfaces (the first ordered fiber layer and the second ordered fiber layer) may be designed with different structures according to the type of tissues contact therewith and the needs of clinical applications.
2. The scaffold for tissue engineering constructed by the invention has a multi-layered structure, and the in vivo degradation time thereof may be adjusted by changing the composition of the polymer material; meanwhile, different three-dimensional porous structures may change the specific surface area and also affect the degradation time of the material; therefore, the degradation time of the scaffold may be controlled according to different application scenarios.
3. The invention employs a near-field direct-write process based on 3D printing, which achieves the printing of ordered linear fiber layers and disordered helical fiber layers by adjusting the printing process parameters of different layers; the printing method is a highly efficient micro-nano scale additive manufacturing technique, with zero additives in the preparation process and no introduction of organic solvents or chemical crosslinking agents.
4. The printing process used in the invention is simple and does not require complex post-processing, which may greatly improve production efficiency and reduce production costs.

### Brief Description of the Drawings

FIG. 1 is a photograph of a biodegradable membrane prepared in Embodiment 1;
FIG. 2 is a scanning electron microscope image of the biodegradable membrane obtained in Embodiment 1 taken from a first ordered fiber layer;
FIG. 3 is a scanning electron microscope image of the biodegradable membrane obtained in Embodiment 1 taken from a second ordered fiber layer;
FIG. 4 includes (a) a photograph of a second composite layer prepared in Embodiment 2, (b) a photograph of a first composite layer prepared in Embodiment 2, and (c) a photograph of the biodegradable membrane prepared in Embodiment 2;
FIG. 5 includes (A) a micrograph of the second composite layer prepared in Embodiment 2, (B) a micrograph of the first composite layer prepared in Embodiment 2, and (C) a micrograph of the biodegradable membrane prepared in Embodiment 2;
FIG. 6 shows the experimental results of culturing cells using a disordered membrane;
FIG. 7 shows the experimental results of culturing cells using the biodegradable membrane prepared in Embodiment 1.

### Detailed Description of the Invention

Taking the preparation of a 10*10mm membrane as an example, the design concept and technical solution of the invention will be further explained.

### Embodiment 1: Printing using direct stacking (continuous printing method)

Specifically:
(1) The material used in this printing is polycaprolactone (PCL). The patterns required for a first ordered fiber layer and a second ordered fiber layer are created using CAD, then exported and converted into Gcode files. The generated Gcode files are then imported into a 3D printing software to generate a printing path.
(2) According to the printing path generated in step (1), with a smooth glass plate as a substrate, first a linear grid structure with a spacing of 100um and a fiber diameter of 5-10um is printed on the substrate to obtain the first ordered fiber layer; the printing process parameters for the layer are a temperature of substrate of 33°C, a substrate-nozzle distance of 2.5mm, a nozzle diameter of 350um, a voltage of 4.7kv, a barrel heating temperature of 90°C, a needle heating temperature of 120°C, a printing speed of 1200mm/min, an air pressure of 1Kpa, and a layer distance compensation of 5um.
(3) Based on the first ordered fiber layer, a helical fiber disorder layer with a pore less than 18µm and a diameter of 5-9µm is printed to obtain an intermediate disorder fiber layer on the first ordered fiber layer; the printing process parameters for the layer are a substrate-nozzle distance of 10mm, a nozzle diameter of 350um, a voltage of 12kv, a barrel heating temperature of 90°C, a needle heating temperature of 120°C, a printing speed of 550mm/min, and an air pressure of 2Kpa.
(4) Finally, the second ordered fiber layer is printed on the intermediate disordered fiber layer to obtain a biodegradable membrane with a three-dimensional ordered porous structure; the second ordered fiber layer is a linear square grid structure with a fiber spacing of 200um and a fiber diameter of 15-20um; the printing process parameters for the layer are a substrate-nozzle distance of 2.6mm, a nozzle diameter of 350um, a voltage of 4.7kv, a barrel heating temperature of 90°C, a needle heating temperature of 120°C, a printing speed of 1200mm/min, an air pressure of 5Kpa, and a layer distance compensation of 10um.

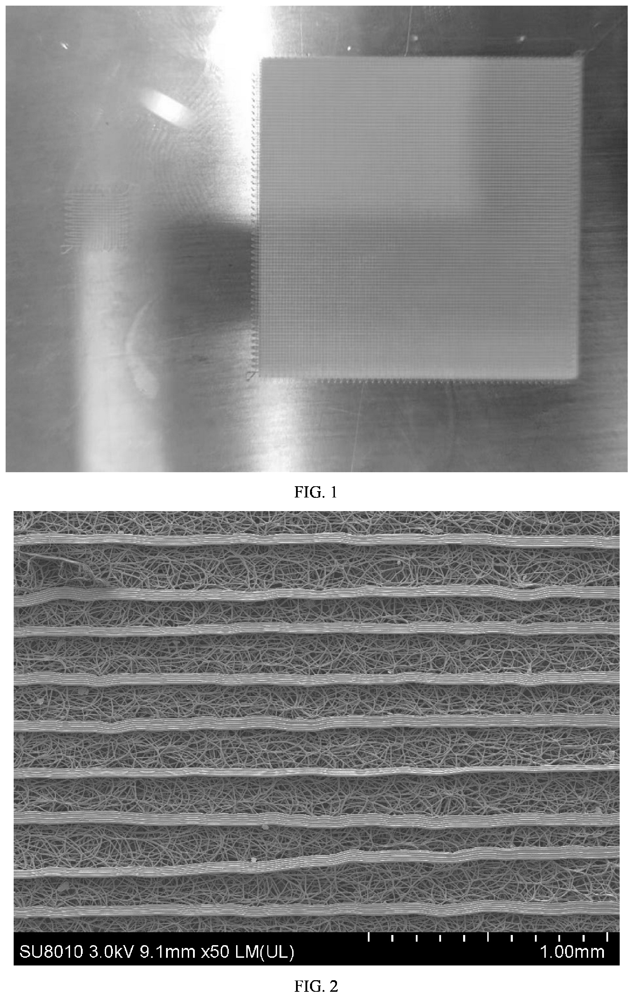

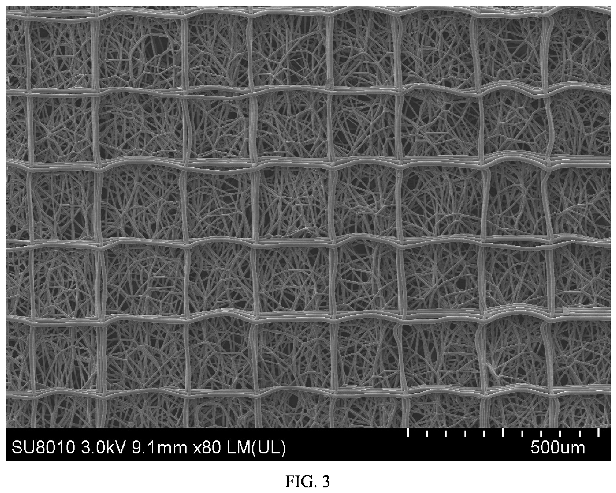

The biodegradable membrane prepared in the embodiment has photographs and scanning electron microscope images thereof shown in FIG. 1 to 3.

### Embodiment 2: Printing using thermal bonding method

Specifically:
(1) The material used in this printing is polycaprolactone. The patterns required for a first ordered fiber layer and a second ordered fiber layer are created using CAD, then exported and converted into Gcode files. The generated Gcode files are then imported into a 3D printing software to generate a printing path.
(2) First, a helical disordered layer with a pore less than 18µm and a diameter of 5-9µm is printed on a smooth glass plate to obtain an intermediate disordered fiber layer; the printing process parameters for the layer are a temperature of the substrate of 33°C, a substrate-nozzle distance of 10mm, a nozzle diameter of 350um, a voltage of 12kv, a barrel heating temperature of 90°C, a needle heating temperature of 120°C, a printing speed of 550mm/min, and an air pressure of 2Kpa.
(3) Then, a linear square grid structure (the second ordered fiber layer) with a gap of 100um and a fiber diameter of 5-10um is printed on the intermediate disordered fiber layer obtained in step (2) to obtain a second composite layer; the printing process parameters for printing the intermediate disordered fiber layer are a substrate-nozzle distance of 2.5mm, a nozzle diameter of 350um, a voltage of 4.7kv, a barrel heating temperature of 90°C, a needle heating temperature of 120°C, a printing speed of 1200mm/min, an air pressure of 1Kpa, and a layer distance compensation of 5um.
(4) A helical disordered layer with a pore of about 150 µm and a diameter of 2-6 µm is printed on another smooth glass plate to obtain a disordered helical fiber layer; the printing process parameters are a temperature of the substrate of 33°C, a substrate-nozzle distance of 10mm, a nozzle diameter of 350um, a voltage of 12kv, a barrel heating temperature of 90°C, a needle heating temperature of 120°C, a printing speed of 1000mm/min, and an air pressure of 1Kpa.
(5) A linear square grid structure (i.e., the first ordered fiber layer) with a fiber spacing of 200um and a fiber diameter of 15-20um is printed on the disordered helical fiber layer obtained in step (4) to obtain a first composite layer; the printing process parameters are a substrate-nozzle distance of 2.6mm, a nozzle diameter of 350um, a voltage of 4.7kv, a barrel heating temperature of 90°C, a needle heating temperature of 120°C, a printing speed of 1200mm/min, an air pressure of 5Kpa, and a layer distance compensation of 10um.
(6) The first and second composite layers printed in steps (3) and (5) are removed, placed in a vacuum drying oven with the disordered helical fiber layer and the intermediate disordered fiber layer facing each other, heated at 52°C for 15 minutes, and subjected to thermal bonding to obtain the biodegradable membrane with a three-dimensional ordered porous structure. During the thermal bonding process, a glass slide is pressed onto the first composite layer and the second composite layer.

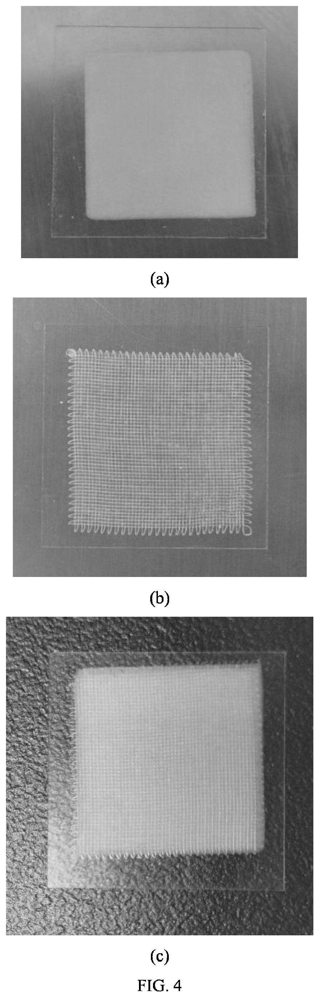

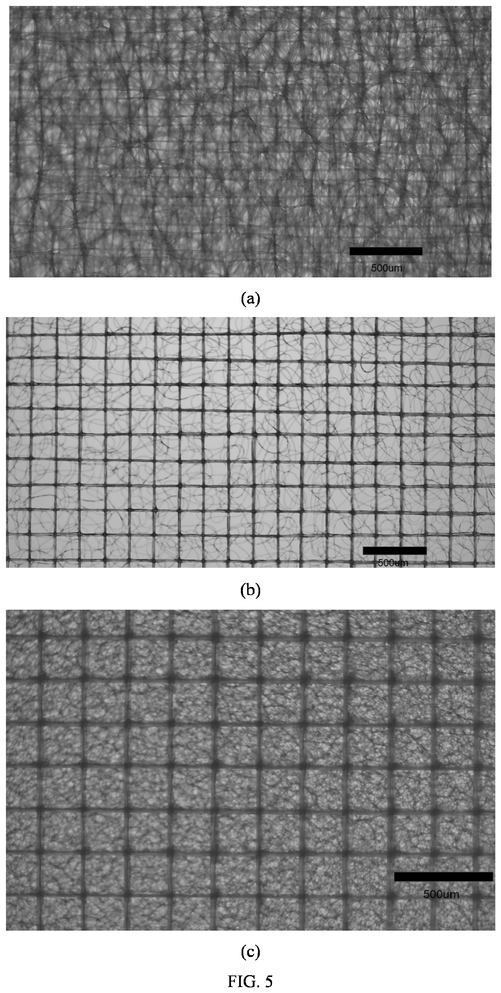

The photographs and micrographs of the first composite layer, the second composite layer, and the biodegradable membrane prepared in the embodiment are shown in FIGS. 4 and 5.

### Performance Test of Product

To evaluate the adhesion and directional growth of gingival fibroblasts (HGF-1) on surfaces of disordered and ordered PCL membranes.

### Preparation of disordered membranes:

(2) A disordered membrane is prepared by printing a helical fiber disordered layer with a pore less than 18 µm and a diameter of 5-9 µm on a smooth glass plate; the printing process parameters are a substrate-nozzle distance of 10mm, a nozzle diameter of 350um, a voltage of 12kv, a barrel heating temperature of 90°C, a needle heating temperature of 120°C, a printing speed of 550mm/min, and an air pressure of 2Kpa.

After a HGF-1 cell suspension is prepared, cells are inoculated onto a surface of the disordered membrane prepared above and the biodegradable membrane obtained in Embodiment 1, respectively. After 24 hours of incubation, fixation is performed with 4% polyoxymethylene for 20 minutes, and then infiltration is performed with 0.5% Triton X-100. Then, under light-protected conditions, the cytoskeleton was stained with Phalloidin (Cytoshelle Inc, USA) working solution for 40 minutes and washed three times with PBS; then the cell nuclei are stained with DAPI (Beyotime, China) working solution for 5 minutes and washed three times with PBS.

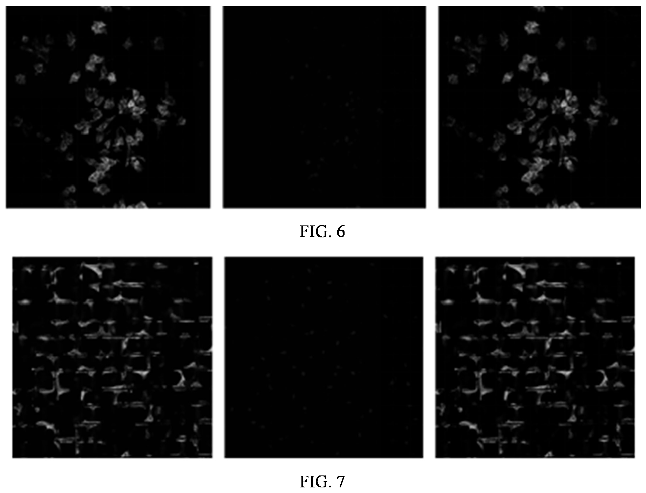

The cell growth on the surface of the disordered membrane and the biodegradable membrane is observed using a laser confocal scanning microscope (CLSM, Leica Microsystems, German). The results are shown in FIGS. 6 and 7, respectively. As shown in FIGS. 6 and 7, HGF-1 cells grow randomly on the surface of the disordered PCL membrane (the disordered membrane), but can grow directionally along fibers on the surface of the ordered layer (the biodegradable membrane). Moreover, the number of cells adhering to the surface of the membrane is greater than that on the surface of the disordered PCL membrane in the same field of view, indicating that the surface of the ordered PCL is more conducive to the early adhesion and directional growth of HGF-1 cells than the structure of the disordered surface.

## Claims

1. A biodegradable membrane with a three-dimensional ordered porous structure, comprising an intermediate disordered fiber layer formed by disorderly stacking helical fibers, and a first ordered fiber layer and a second ordered fiber layer disposed on both sides of the intermediate disordered fiber layer, the first and second ordered fiber layers being each independently formed by stacking single and multiple layers of linear fibers and having an ordered structure;
a first ordered fiber layer material, an intermediate disordered fiber layer material, and a second ordered fiber layer material are each independently selected from a biodegradable material.

2. The biodegradable membrane with a three-dimensional ordered porous structure according to claim 1, wherein the biodegradable material is one or more copolymers or mixtures of polylactic acid, polyglycolic acid, polycaprolactone, polylactic-co-hydroxyacetic acid copolymer, polyethylene glycol, polyurethane, polyhydroxybutyrate, polyhydroxyvalerate, polydioxanone, polytrimethylene carbonate, polybutylene succinate, chitosan and derivatives thereof, carboxymethyl cellulose, gelatin, hyaluronic acid, collagen, alginate, gum, whey protein and derivatives thereof.

3. The biodegradable membrane with a three-dimensional ordered porous structure according to claim 1, wherein fiber spacings of the first ordered fiber layer and the second ordered fiber layer are each independently selected from 1 to 500 µm.

4. A preparation method for the biodegradable membrane with a three-dimensional ordered porous structure according to any one of claims 1 to 3, comprising: using near-field direct-write 3D printing technology to print the first ordered fiber layer and the second ordered fiber layer with parameters of a nozzle-substrate distance of 0.5~5mm, an extrusion pressure of 0.1KPa~1MPa and a voltage of 1~6KV;
printing the intermediate disordered fiber layer with parameters of the nozzle-substrate distance of 1~50mm, the extrusion pressure of 0.5~50KPa and the voltage of 1~30KV.

5. The preparation method for the biodegradable membrane with a three-dimensional ordered porous structure according to claim 4, comprising printing using a continuous printing method, which comprises:
sequentially printing the first ordered fiber layer, the intermediate disordered fiber layer, and the second ordered fiber layer on a substrate to obtain the biodegradable membrane with a three-dimensional ordered porous structure.

6. The preparation method for the biodegradable membrane with a three-dimensional ordered porous structure according to claim 4, comprising printing using a thermal bonding method, which specifically comprises steps of:
sequentially printing a disordered helical fiber layer and the first ordered fiber layer on a substrate to obtain a first composite layer;
sequentially printing the intermediate disordered fiber layer and the second ordered fiber layer on another substrate to obtain a second composite layer;
thermally bonding the disordered helical fiber layer of the first composite layer and the intermediate disordered fiber layer of the second composite layer to obtain the biodegradable membrane with a three-dimensional ordered porous structure;
wherein when the disordered helical fiber layer is printed, the nozzle-substrate distance is controlled to be 1~50mm, the extrusion pressure to be 0.5~50KPa, and the voltage to be 1~30KV.

7. The preparation method for the biodegradable membrane with a three-dimensional ordered porous structure according to claim 6, wherein the thermal bonding temperature is 0.2 to 0.9 times a melting point of a material of the intermediate disordered fiber layer.

8. The preparation method for the biodegradable membrane with a three-dimensional ordered porous structure according to claim 6, wherein a fiber diameter of the disordered helical fiber layer is 0.01 to 1 times the fiber diameter of the first ordered fiber layer, with a fiber path being the same as that of the first ordered fiber layer.

9. The preparation method for the biodegradable membrane with a three-dimensional ordered porous structure according to claim 4, wherein a roughness of the substrate is Rₐ≤1.6µm.

10. An application of the biodegradable membrane with a three-dimensional ordered porous structure according to any one of claims 1 to 3 as a scaffold for tissue engineering.
